# EUROPÄISCHE PATENTSCHRIFT

(11) **EP 2 627 259 B1**
(45) Veröffentlichungstag und Bekanntmachung des Hinweises auf die Patenterteilung: **15.03.2017**
(21) Anmeldenummer: 11761474.3
(22) Anmeldetag: 15.09.2011
(51) Int. Cl.: A61B 17/00, B05B 7/04

(54) **MEDIZINISCHER SPRÜHKOPF MIT DRUCKGASUNTERSTÜTZUNG**
MEDICAL SPRAYHEAD WITH PRESSURIZED GAS
TÊTE DE PULVÉRISATION MEDICAL AVEC GAZ SOUS PRESSION

(30) Priorität: 15.10.2010 CH 16982010
(43) Veröffentlichungstag der Anmeldung: 21.08.2013
(73) Patentinhaber: Medmix Systems AG, 6343 Rotkreuz (CH)
(72) Erfinder: GRETER, Andy, CH-6340 Baar (CH)
(74) Vertreter: Detken, Andreas
(86) Internationale Anmeldenummer: PCT/CH2011/000217
(87) Internationale Veröffentlichungsnummer: WO 2012/048434

(56) Entgegenhaltungen:
- WO-A1-2007/000066
- US-A- 4 940 185
- US-A1- 2007 005 007

## Beschreibung

### TECHNISCHES GEBIET

Die vorliegende Erfindung betrifft einen Sprühkopf zum Anschliessen an mindestens ein Reservoir mit einer fliessfähigen Substanz und zum Versprühen dieser Substanz unter Anwendung von Druckgas, z.B. von Druckluft oder von einem anderen komprimierten Gas wie Kohlendioxid.

### STAND DER TECHNIK

Sprühköpfe zum Versprühen einer oder mehrerer Substanzen mit Druckgasunterstützung finden unter anderem im medizinischen Bereich Verwendung, z.B. zum Versprühen eines medizinischen Zweikomponentenklebers auf der Basis von Fibrinogen und Thrombin. Derartige sogenannte Fibrinkleber werden in der Medizin z.B. zum Verschliessen von Läsionen, zum Stoppen von Blutungen (Hämostase) oder als Gewebekleber zum Befestigen von Hauttransplantaten eingesetzt. Ein solcher Sprühkopf wird zum Gebrauch an eines oder mehrere Reservoirs mit den zu versprühenden Substanzen angeschlossen, z.B. an eine Einfachspritze mit dem schon vorgemischten Klebemittel oder an eine Doppelspritze mit den Komponenten des Klebemittels. Um die Substanzen zu versprühen, wird der Sprühkopf einerseits mit Druckgas beaufschlagt, um einen Druckgasstrom zu erzeugen; andererseits trägt der Benutzer die Substanzen aus den Reservoirs aus, um sie in den Druckgasstrom einzubringen und dadurch zu zerstäuben.

Ein solcher Sprühkopf ist z.B. aus der WO 2010/048734 bekannt. Die zu versprühenden Substanzen werden in einem zentralen Bereich des Sprühkopfes durch einen oder mehrere Fluidkanäle zu düsenartigen Fluidauslässen an einer Spitze des Sprühkopfes geführt. Ein Ringkanal für ein Druckgas umgibt die Fluidkanäle und mündet in mehrere Gasauslässe, die leicht zurückversetzt zu den Fluidauslässen angeordnet sind. Im Betrieb tritt durch die Gasauslässe ein Gasstrom aus. Dieser trifft auf die aus den Fluidauslässen austretenden Substanzen, zerteilt diese in feine Tröpfchen und transportiert den entstehenden Sprühnebel zu einem vorgesehenen Applikationsbereich, z.B. zu einer zu behandelnden Wunde eines Patienten.

Aus dem Stand der Technik sind eine Vielzahl weiterer Bauformen von Sprühköpfen bekannt. So wurde z.B. vorgeschlagen, das Druckgas im Sprühkopf zentral zuzuführen, während die zu versprühenden Substanzen durch einen Ringraum oder durch dezentral angeordnete Fluidkanäle zugeführt werden. Ein Beispiel für eine solche Bauform ist in US 5,605,541 angegeben. Des weiteren sind Sprühköpfe bekannt, bei denen die zu versprühenden Substanzen in einer Mischkammer mit dem Druckgas vermischt werden, bevor der so schon vorgemischte Sprühnebel aus einer Auslassdüse des Sprühkopfes austritt. Beispiele hierfür finden sich in US 2003/0209612 oder US 2009/0108091. Für endoskopische, laparoskopische oder andere minimalinvasive Anwendungen sind Sprühköpfe bekannt, die ein langgestrecktes Rohr aufweisen, das durch eine Körperöffnung hindurch ins Körperinnere einführbar ist und durch welches hindurch die Substanzen und das Druckgas zugeführt werden. Beispiele hierfür finden sich in US 2009/0209916 oder US 2007/0005007. Dazu ist es bekannt, die zu versprühenden Substanzen und das Druckgas durch separate Leitungen zu einem distalen Ende des Sprühkopfes zu führen, wie dies z.B. auch in US 5,810,885 beschrieben ist.

Unabhängig von der konkreten Bauform besteht bei druckgasunterstützten Sprühköpfen die Gefahr, dass das Druckgas bei einem zu hohen Gasdruck in Blutgefässe im Applikationsbereich eindringt und dadurch eine Gasembolie verursacht. Auch unabhängig von dieser konkreten Gefahr kann ein zu hoher Gasdruck zu unerwünschten Ergebnissen führen, z.B. zu einem ungleichmässigen Sprühbild oder zu einer unkontrollierten Verteilung der zu versprühenden Substanzen über den vorgesehenen Applikationsbereich hinaus. Schliesslich kann es auch vorkommen, dass die zu versprühenden Substanzen die Gasaustrittskanäle verstopfen und dass in der Folge der Gasdruck im Sprühkopf plötzlich ansteigt. In ungünstigen Fällen kann dadurch das Druckgas ungewollt in die Fluidkanäle und von dort in die Fluidreservoirs gelangen, in denen die Substanzen aufgenommen sind. Wenn die Fluidreservoirs als Spritzen ausgebildet sind, kann dies dazu führen, dass die

Spritzenkolben zurückgepresst werden. Dies ist in hohem Masse unerwünscht.

Um den Gasdruck am Sprühkopf zu begrenzen, ist es bekannt, zwischen der Druckgasquelle und dem Sprühkopf einen Gasregler vorzusehen, der den Druck des Gases, das zum Sprühkopf gelangt, auf einen vorgegebenen Maximalwert begrenzt. So ist z.B. in US 2007/0005007 ein Sprayapplikator angegeben, in dessen Gasregler ein Überdruckventil vorgesehen ist. Allerdings besteht bei derartigen Gasreglern immer die Gefahr einer Fehlfunktion oder einer Fehlbedienung. Fehlbedienungen können insbesondere dann auftreten, wenn mehrere verschiedene Arten von Sprühköpfen alternativ an den selben Gasregler anschliessbar sind. In diesem Fall kann der vorgesehene Betriebsdruck von Sprühkopf zu Sprühkopf unterschiedlich sein, und es besteht die Gefahr, dass das Bedienpersonal beim Wechsel von einem Sprühkopf, der mit einem höheren Betriebsdruck betrieben wird, zu einem Sprühkopf, der mit einen geringeren Betriebsdruck zu betreiben wäre, vergisst, den Gasregler entsprechend herunterzuregeln.

In der US 6,135,358 wird eine druckgasunterstützte Vorrichtung zum Spülen der Nasenhöhlen vorgeschlagen, die mit einem Regelventil zur Regelung des Gasdrucks versehen ist. Diese Vorrichtung ist bauartbedingt lediglich für das Spülen von Nasenhöhlen geeignet und eignet sich nicht zum Versprühen eines medizinischen Klebemittels oder von dessen Komponenten. Sie eignet sich insbesondere nicht dazu, an ein oder mehrere Reservoirs mit den zu versprühenden Substanzen angeschlossen zu werden.

Aus US 4,940,185 ist eine Sprühpistole bekannt, die zum Versprühen eines Sprühguts, z.B. von Farbe, aus einer an die Sprühpistole angeschlossenen Flasche vorgesehen ist. Druckgas wird zu einer Sprühdüse geführt. Dasselbe Druckgas wird auch in die Flasche eingeleitet, um in der Flasche einen Überdruck aufzubauen und dadurch das Sprühgut aus der Flasche zur Sprühdüse zu transportieren. Um zu verhindern, dass sich in der Flasche ein übermässiger Druck aufbaut, ist ein Sicherheitsventil in der Zuleitung des Druckgases zur Flasche vorgesehen, nicht aber in der Zuleitung des Druckgases zur Düse. Diese Sprühpistole ist offensichtlich nicht für medizinische Anwendungen vorgesehen und verhindert auch nicht, dass das Druckgas mit einem unerwünscht hohen Druck aus der Sprühdüse austreten kann.

### DARSTELLUNG DER ERFINDUNG

Es ist daher eine Aufgabe der vorliegenden Erfindung, einen Sprühkopf zum Versprühen mindestens einer fliessfähigen Substanz aus einem externen Reservoir mit Hilfe eines Druckgases anzugeben, bei dem verhindert wird, dass der Druck des Druckgases im Sprühkopf über einen bestimmten Maximalwert hinaus ansteigt.

Diese Aufgabe wird durch einen Sprühkopf mit den Merkmalen des Anspruchs 1 gelöst. Weitere Ausführungsformen sind in den abhängigen Ansprüchen angegeben.

Es wird also ein Sprühkopf zum Versprühen mindestens einer fluiden Substanz unter Einsatz eines Druckgases vorgeschlagen, welcher aufweist:
mindestens einen Fluidanschluss, um den Sprühkopf mit mindestens einem Fluidreservoir für die zu versprühende Substanz zu verbinden;
mindestens einen Fluidkanal, der den Fluidanschluss mit mindestens einem Fluidauslass verbindet;
einen Druckgasanschluss, um den Sprühkopf mit einer Druckgasquelle zu verbinden; und
mindestens einen Druckgaskanal, der den Druckgasanschluss mit mindestens einem Druckgasauslass verbindet,
wobei der Fluidauslass und der Druckgasauslass derart zueinander angeordnet sind, dass im Betrieb aus dem Druckgasauslass austretendes Druckgas auf eine aus dem Fluidauslass austretende Substanz trifft, um die Substanz zu versprühen.

Der Sprühkopf zeichnet sich dadurch aus, dass er ein Überdruckventil aufweist, welches dazu ausgebildet ist, Druckgas aus dem Druckgaskanal abzuleiten, wenn das Druckgas einen Gasdruck aufweist, der einen Schwellendruck überschreitet.

Dadurch kann direkt am Sprühkopf selbst sichergestellt werden, dass ein bestimmter Maximaldruck (der Schwellendruck) am Druckgasauslass nicht wesentlich überschritten wird. Auf diese Weise wird wirksam verhindert, dass die Substanz mit einem zu hohen Gasdruck versprüht wird. Das Embolierisiko beim Einsatz eines solchen Sprühkopfes an offenem Körpergewebe wird so merklich vermindert. Falls der Sprühkopf zusammen mit einem externen Gasregler verwendet wird, verhindert der vorgeschlagene Sprühkopf wirksam negative Folgen von Fehlbedienungen oder Fehlfunktionen des Gasreglers. Indem das Überdruckventil am Sprühkopf selbst vorgesehen ist, haben Störungen, die möglicherweise zwischen der Druckgasquelle bzw. dem Gasregler und dem Sprühkopf auftreten können, keinen Einfluss auf die wirksame Begrenzung des Gasdrucks unmittelbar am Sprühkopf.

Das Überdruckventil ist vorzugsweise an einem Bereich des Druckgaskanals vorgesehen, der bezüglich der Strömungsrichtung des Druckgases stromaufwärts vom Druckgasauslass angeordnet ist. Dadurch ist die Funktion des Überdruckventils selbst dann sichergestellt, falls die zu versprühende Substanz den Bereich des Druckgasauslasses verstopfen sollte.

Andererseits ist das Überdruckventil aber auch bevorzugt an einem Bereich des Druckgaskanals angeordnet, der bezüglich der Strömungsrichtung des Druckgases stromabwärts vom Druckgasanschluss angeordnet ist. Insbesondere ist es also bevorzugt, wenn das Überdruckventil nicht an einer separat mit dem Druckgasanschluss verbundenen Leitung ausgebildet ist, sondern eben gerade an jenem Teil des Druckgaskanals, der auch tatsächlich vom Druckgas auf seinem Weg zwischen Druckgasanschluss und Druckgasauslass durchströmt wird. Auf diese Weise ist sichergestellt, dass das Ventil mit dem Druck desjenigen Gases beaufschlagt ist, das zum Druckgasauslass hin strömt, und dass nicht etwa eine Verengung oder Verstopfung einer separaten Ventilzuleitung den Druck am Ventil selbst unerwünscht beeinflussen kann.

Für den Ort, an dem das Überdruckventil am Sprühkopf vorgesehen ist, gibt es abhängig von der konkreten Bauform des Sprühkopfes verschiedene Möglichkeiten. So kann das Überdruckventil z.B. an einem Bereich des Druckgaskanals vorgesehen sein, der den Fluidkanal wenigstens teilweise umgibt. In einigen Ausgestaltungen eines Sprühkopfes kann der Sprühkopf ein statisches Mischelement aufweisen, das im Fluidkanal angeordnet ist. Um einen kompakten Sprühkopf zu schaffen, kann dann das Überdruckventil an einem Bereich des Druckgaskanals vorgesehen sein, der denjenigen Bereich des Fluidkanals wenigstens teilweise umgibt, in dem das statische Mischelement angeordnet ist.

In einigen Bauformen kann der Sprühkopf einen eine Vorrichtungslängsachse definierenden Basiskörper und einen vom Basiskörper abstehenden Gaszufuhrstutzen aufweisen, an dem der Druckgasanschluss ausgebildet ist. Der Gaszufuhrstutzen kann insbesondere rohrförmig sein. Der Gaszufuhrstutzen verläuft dann vorzugsweise gewinkelt, insbesondere um ca. 30°-90° gewinkelt, bevorzugt um ca. 50°-80° gewinkelt, zur Vorrichtungslängsachse, wobei sein einlassseitiges Ende vom Druckgasauslass am Basiskörper wegweist. In vorteilhaften Ausgestaltungen ist das Überdruckventil am Basiskörper angeordnet und weist einen Ventilauslasskanal auf, der im Wesentlichen parallel zum Gaszufuhrstutzen verläuft, wobei der Ventilauslasskanal mit seinem auslassseitigen Ende ebenfalls vom Druckgasauslass weg weist. Alternativ kann das Überdruckventil aber auch am Gaszufuhrstutzen angeordnet sein. Durch diese Massnahmen stört das Überdruckventil möglichst wenig bei der Anwendung des Sprühkopfes, und das Druckgas, das durch das Überdruckventil entweicht, gelangt mit geringerer Wahrscheinlichkeit zum Applikationsbereich.

Rein beispielhaft kann der Sprühkopf grundsätzlich entsprechend der schon erwähnten WO 2010/048734 aufgebaut sein, deren Inhalt hierin durch Verweis aufgenommen wird. Der Sprühkopf kann insbesondere eine innere Hülse aufweisen, die in jenem Dokument als Gehäuse bezeichnet wird und in welcher der mindestens eine Fluidkanal ausgebildet ist. Der Sprühkopf kann dann ausserdem eine äussere Hülse aufweisen, die in jenem Dokument als Kappe bezeichnet wird und die die innere Hülse mindestens teilweise umgibt. Die äussere Hülse und die innere Hülse begrenzen gemeinsam zumindest einen Abschnitt des Druckgaskanals. Dazu kann die äussere Hülse in einem proximalen Bereich dichtend mit der inneren Hülse verbunden sein, während ihr distales offenes Ende gemeinsam mit der inneren Hülse mehrere ringförmig angeordnete, einen oder mehre Fluidauslässe ringförmig umgebende Gasauslässe begrenzt. Eine besonders einfache und elegante Bauform resultiert, wenn bei einem solchen Aufbau sowohl der Druckgasanschluss als auch das Überdruckventil an der äusseren Hülse ausgebildet sind. Die äussere Hülse definiert hier mit ihrer Zylinderachse eine Längsachse. Das Überdruckventil weist dann vorteilhaft einen Ventilauslasskanal auf, dessen Längsrichtung zur Längsachse der äusseren Hülse einen Winkel zwischen 30° und 90°, bevorzugt 50° bis 80° einnimmt, wobei der Ventilauslasskanal mit seinem auslassseitigen Ende vom Druckgasauslass weg weist.

Es sind aber auch völlig andere Bauformen möglich. So sind auch langgestreckte Bauformen mit einem langgestreckten distalen Rohr für endoskopische oder minimalinvasive Anwendungen möglich. Bei solchen Ausgestaltungen ist es bevorzugt, wenn das Überdruckventil entfernt vom distalen Ende in einem proximalen Endbereich des Rohrs oder proximal von diesem Rohr angeordnet ist.

Der Sprühkopf kann spezifisch dazu ausgebildet sein, ein medizinisches Produkt (z.B. ein Medikament oder ein medizinisches Klebemittel) oder Komponenten eines solchen Produkts zu versprühen. Aus diesem Einsatzzweck ergeben sich verschiedene Anforderungen an Bauform, Materialwahl und Dimensionierung des Sprühkopfes. So sollte der Sprühkopf für diesen Einsatzzweck insbesondere aus pharmakologisch und toxikologisch unbedenklichen ("medical grade") Materialien (geeigneter Kunststoff und/oder geeignetes Metall wie Edelstahl oder Titan) gefertigt sein. Die Fluidanschlüsse können für diesen Einsatzzweck als Luerverbindungen oder als spezielle Systemverbindungen für passende Systembehälter ausgestaltet sein. Auch der Gaseinlass kann in Form einer Luerverbindung ausgebildet sein. Luerverbindungen sind genormte konische Steckverbindungen, die optional mit einer Sicherungshülse (Luer-Lock) gesichert sein können. In der Regel sollte ein Sprühkopf für medizinische Anwendungen möglichst kleine laterale Abmessungen (d.h. Abmessungen quer zur Längsachse des Sprühkopfes) aufweisen, die einige Zentimeter nicht übersteigen, insbesondere ca. 5 cm nicht übersteigen, um dem medizinischen Fachpersonal bei der Anwendung möglichst wenig im Weg zu sein. Im Gegensatz hierzu sind insbesondere handelsübliche Sprühpistolen zum Versprühen von Farbe in konstruktiver Hinsicht, aber auch aus Gründen der Materialwahl und Dimensionierung offensichtlich nicht für medizinische Zwecke geeignet und weisen in der Regel erheblich grössere laterale Abmessungen auf.

Insbesondere kann der Sprühkopf dazu ausgebildet sein, zwei oder mehr Komponenten eines Mehrkomponentensystems, z.B. die Komponenten eines Medikaments oder eines medizinischen Klebemittels, separat zum Sprühkopf zuzuführen und erst im Sprühkopf oder nach Verlassen des Sprühkopfes miteinander zu mischen. Dazu kann der Sprühkopf einen ersten Fluidanschluss und einen zweiten Fluidanschluss aufweisen, wobei jeder der Fluidanschlüsse dazu ausgebildet ist, den Sprühkopf mit jeweils einem Fluidreservoir für eine zu versprühende Komponente zu verbinden. Der Sprühkopf weist dann einen ersten Fluidkanal auf, der den ersten Fluidanschluss mit einem ersten Fluidauslass verbindet, sowie einen zweiten Fluidkanal, der den zweiten Fluidanschluss mit einem zweiten Fluidauslass verbindet. Der erste Fluidauslass, der zweite Fluidauslass und der Druckgasauslass sind dann vorzugweise derart zueinander angeordnet, dass sich im Betrieb aus dem Druckgasauslass austretendes Druckgas mit den beiden aus den Fluidauslässen austretenden Komponenten vermischt, um die Komponenten in feine Tröpfchen zu zerteilen und gemeinsam zu versprühen. Insbesondere können dazu die Fluidauslässe bezüglich der Strömungsrichtung des Druckgases stromabwärts vom Druckgasauslass oder den Druckgasauslässen angeordnet sein.

Während die Erfindung nicht auf eine bestimmte Art von Überdruckventil beschränkt ist, sind bestimmte Bauformen aufgrund ihrer Einfachheit, Robustheit und kostengünstigen Fertigung bevorzugt. So kann das Überdruckventil insbesondere einen elastischen Verschlusskörper umfassen, der derart an einer Ventilbasis befestigt ist, dass er einen Ventilauslasskanal bzw. eine Ventilöffnung aufgrund seiner eigenen Elastizität verschliesst, solange der Gasdruck den Schwellendruck nicht überschreitet. Der Verschlusskörper kann z.B. eine mittig an der Ventilbasis befestigte elastische Scheibe sein. Alternativ kann das Ventil auch als sogenanntes Schirmventil oder Pilzventil (engl. "umbrella valve") ausgebildet sein. In diesem Fall ist der Verschlusskörper schirmförmig ausgebildet. Bei Überschreiten des Schwellendrucks hebt in solchen Ausgestaltungen die Druckkraft jeweils den Verschlusskörper gegen die elastische Rückstellkraft des Verschlusskörpers selbst vom Ventilauslasskanal ab.

Alternativ kann das Überdruckventil einen starren, beweglichen Verschlusskörper umfassen, der derart federbelastet ist, dass er einen Ventilauslasskanal verschliesst, solange der Gasdruck den Schwellendruck nicht überschreitet. Beim Verschlusskörper kann es sich in diesem Fall z.B. um eine Verschlusskugel oder eine Verschlussscheibe handeln.

Auch ist es z.B. möglich, dass das Überdruckventil eine elastische Manschette umfasst, die einen rohrförmigen Abschnitt des Sprühkopfes aussenseitig umgibt und aufgrund ihrer Elastizität mindestens einen im rohrförmigen Abschnitt vorgesehenen Ventilauslasskanal verschliesst, solange der Gasdruck den Schwellendruck nicht überschreitet.

Die vorliegende Erfindung bezieht sich ausserdem auch auf eine komplette Sprühvorrichtung zum Versprühen eines medizinischen Produkts (z.B. eines Medikaments oder medizinischen Klebemittels). Diese weist auf:
mindestens ein Fluidreservoir mit einer darin aufgenommenen, fluiden medizinischen Substanz;
einen mit dem Fluidreservoir verbindbaren Sprühkopf der vorstehend dargestellten Art; und
eine Austrageinrichtung zum Austragen der Substanz durch den Sprühkopf hindurch.

Das Fluidreservoir kann z.B. nach Art eines zylindrischen Spritzenkörpers mit einem Auslass und einem im Spritzenkörper beweglichen Kolben ausgestaltet sein, und die Austrageinrichtung kann dann eine Kolbenstange umfassen, um den Kolben im Spritzenkörper in die distale Richtung vorzuschieben und auf diese Weise die Substanz aus dem Fluidreservoir auszutragen.

Die Sprühvorrichtung kann auch zum Versprühen von mindestens zwei Komponenten eines medizinischen Produkts (z.B. der Komponenten eines Medikaments oder eines medizinischen Klebemittels) ausgebildet sein. In diesem Fall weist die Sprühvorrichtung auf:
mindestens zwei Fluidreservoirs mit jeweils einer darin aufgenommenen Komponente des medizinischen Produkts;
einen mit den Reservoirs verbindbaren Sprühkopf nach einem der vorhergehenden Ansprüche, wobei der Sprühkopf mindestens zwei Fluidanschlüsse zum Verbinden des Sprühkopfs mit jeweils einem Auslass der Fluidreservoirs und mindestens zwei Fluidkanäle aufweist, die die Fluidanschlüsse mit Fluidauslässen des Sprühkopfs verbinden; und
eine Austrageinrichtung zum Austragen der Komponenten durch den Sprühkopf hindurch.

Die Fluidreservoirs können hier z.B. nach Art einer Doppelspritze, Mehrfachspritze, Doppelkartusche oder Mehrfachkartusche angeordnet sein, mit zwei oder mehr parallel angeordneten, zylindrischen Behältern mit jeweils einem Auslass und mit jeweils einem verschiebbaren Kolben, wobei jeder dieser Behälter ein Fluidreservoir begrenzt. Die Austrageinrichtung kann dann zwei oder mehr Kolbenstangen umfassen, die optional miteinander verbunden sein können, um die Kolben in die distale Richtung vorzuschieben und auf diese Weise die Komponenten aus den Fluidreservoirs auszutragen.

Die vorliegende Erfindung bezieht sich schliesslich auch auf die Verwendung eines Sprühkopfes oder einer Sprühvorrichtung der vorstehend angegebenen Art zum Versprühen eines medizinischen Produkts (insbesondere eines Medikaments oder eines medizinischen Klebemittels) oder von Komponenten eines solchen medizinischen Produkts, insbesondere eines Fibrinklebers bzw. der Komponenten eines Fibrinklebers. In einem solchen Verfahren zum Versprühen eines medizinischen Produkts bzw. seiner Komponenten werden insbesondere die folgenden Schritte ausgeführt:
Bereitstellen des medizinischen Produkts bzw. seiner Komponenten in jeweils einem Reservoir;
Verbinden des Fluidanschlusses bzw. der Fluidanschlüsse des Sprühkopfes mit dem Auslass des Reservoirs bzw. mit den Auslässen der Reservoirs;
Zuführen von Druckgas zum Druckgasanschluss des Sprühkopf, so dass das Druckgas durch den Druckgasauslass bzw. die Druckgasauslässe ausströmt;
Austragen des medizinischen Produkts bzw. der Komponenten durch den Fluidauslass bzw. die Fluidauslässe, um das Produkt bzw. die Komponenten mit Hilfe des Druckgases zu versprühen; und
Ablassen von Druckgas durch das Überdruckventil, falls das Überdruckventil öffnet.

In einem solchen Verfahren kann vorgesehen sein, den Sprühvorgang abzubrechen, falls Druckgas aus dem Überdruckventil ausströmt, da dies eine Fehlbedienung oder Fehlfunktion anzeigt. Insbesondere wenn das Produkt ein medizinisches Klebemittel ist, kann auf diese Weise das Risiko einer Gasembolie weiter vermindert werden.

### KURZE BESCHREIBUNG DER ZEICHNUNGEN

Bevorzugte Ausführungsbeispiele der Erfindung werden im Folgenden anhand der

Zeichnungen beschrieben, die lediglich zur Erläuterung dienen und nicht einschränkend auszulegen sind. In den Zeichnungen zeigen:
- Fig. 1: eine perspektivische Ansicht einer Spritze mit einem daran angebrachten Sprühkopf;
- Fig. 2: eine Draufsicht der Spritze und des Sprühkopfes der Fig. 1;
- Fig. 3: einen vergrösserten Ausschnitt aus einem Längsschnitt der Spritze und des Sprühkopfes in der Ebene A-A der Fig. 2;
- Fig. 4: eine perspektivische Ansicht eines Sprühkopfes gemäss einem ersten Ausführungsbeispiel der vorliegenden Erfindung;
- Fig. 5: eine Frontansicht des Sprühkopfes der Fig. 4;
- Fig. 6: einen zentralen Längsschnitt des Sprühkopfes in der Ebene B-B der Fig. 5;
- Fig. 7: eine perspektivische Explosionsdarstellung des Sprühkopfes der Fig. 4;
- Fig. 8: eine perspektivische Ansicht eines Sprühkopfes gemäss einem zweiten Ausführungsbeispiel der vorliegenden Erfindung;
- Fig. 9: einen zentralen Längsschnitt des Sprühkopfes der Fig. 8;
- Fig. 10: eine perspektivische Explosionsdarstellung des Sprühkopfes der Fig. 8;
- Fig. 11: eine perspektivische Ansicht eines Sprühkopfes gemäss einem dritten Ausführungsbeispiel der vorliegenden Erfindung;
- Fig. 12: einen zentralen Längsschnitt des Sprühkopfes der Fig. 11;
- Fig. 13: eine perspektivische Explosionsdarstellung des Sprühkopfes der Fig. 11;
- Fig. 14: eine perspektivische Ansicht eines Sprühkopfes gemäss einem vierten Ausführungsbeispiel der vorliegenden Erfindung;
- Fig. 15: einen zentralen Längsschnitt des Sprühkopfes der Fig. 14;
- Fig. 16: eine perspektivische Explosionsdarstellung des Sprühkopfes der Fig. 14;
- Fig. 17: eine perspektivische Ansicht eines Sprühkopfes gemäss einem fünften Ausführungsbeispiel der vorliegenden Erfindung;
- Fig. 18: einen zentralen Längsschnitt des Sprühkopfes der Fig. 17;
- Fig. 19: eine perspektivische Explosionsdarstellung des Sprühkopfes der Fig. 17;
- Fig. 20: eine perspektivische Ansicht eines Sprühkopfes gemäss einem sechsten Ausführungsbeispiel der vorliegenden Erfindung;
- Fig. 21: eine Seitenansicht des Sprühkopfes der Fig. 20;
- Fig. 22: einen zentralen Längsschnitt des Sprühkopfes in der Ebene C-C der Fig. 21;
- Fig. 23: eine Ansicht des Sprühkopfes der Fig. 20 vom proximalen Ende her;
- Fig. 24: einen zentralen Längsschnitt des Sprühkopfes in der Ebene D-D der Fig. 23; sowie
- Fig. 25: eine Detailansicht der Fig. 24 im Bereich E.

### BESCHREIBUNG BEVORZUGTER AUSFÜHRUNGSFORMEN

In diesem Dokument werden Richtungsangaben wie folgt verwendet: Als distale Richtung wird diejenige Richtung bezeichnet, zu der hin die Abgabe der zu versprühenden Substanzen erfolgt. Die proximale Richtung ist die dazu entgegengesetzte Richtung.

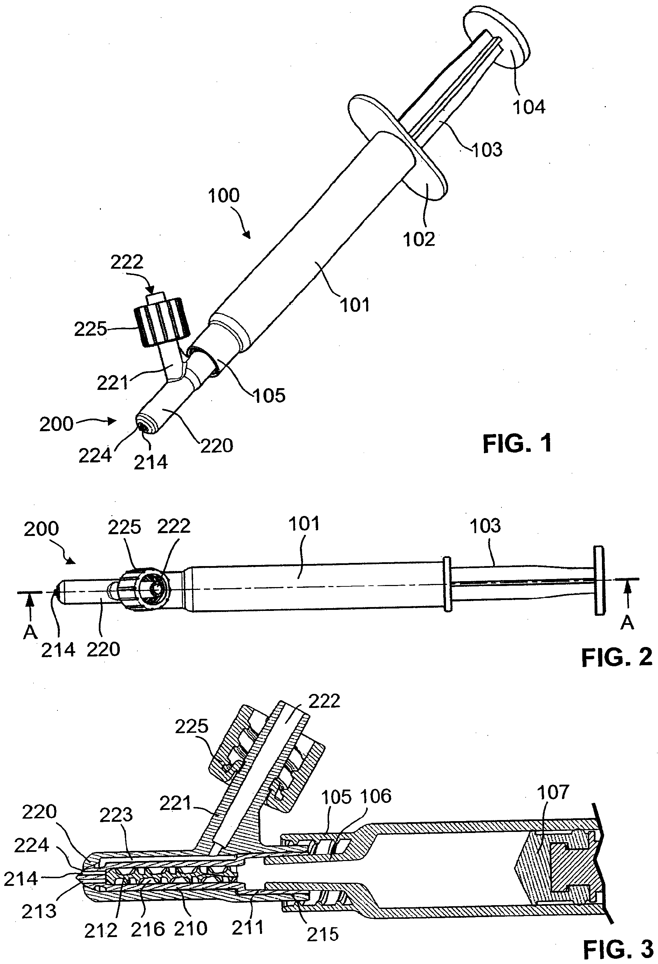

In den Figuren 1 bis 3 ist eine Spritze 100 mit einem daran angebrachten, nicht erfindungsgemäss ausgestalteten Sprühkopf 200 dargestellt, der hier zur Erläuterung einer möglichen grundsätzlichen Bauform eines Sprühkopfes dargestellt ist. Die Spitze 100 umfasst einen zylindrischen Spritzenkörper 101, an dessen proximalem Ende zwei gegenüberliegende, radial abstehende Halteflansche 102 ausgebildet sind. Im Inneren des Spritzenkörpers begrenzt ein verschiebbarer Kolben 107 gemeinsam mit dem Spritzenkörper ein Fluidreservoir. Der Kolben 107 lässt sich im Spritzenkörper mittels einer Kolbenstange 103 mit Daumenauflage 104 in die distale Richtung vorschieben, um ein fluides Produkt durch einen Auslassbereich 106 aus dem Fluidreservoir auszustossen.

Der Sprühkopf 200 umfasst eine innere Hülse 210, die von einer äusseren Hülse 220 umgeben ist. Die innere Hülse 210 und die äussere Hülse 220 bilden gemeinsam einen Basiskörper des Sprühkopfes. Die innere Hülse 210 begrenzt einen zentralen Fluidkanal 216, welcher sich von einem aufgeweiteten proximalen Fluidanschluss 211 zu einem oder mehreren düsenartigen distalen Fluidauslässen 214 erstreckt. In den Fluidkanal ist ein statisches Mischelement 212 mit einer Mehrzahl hintereinander angeordneter, helikaler Mischblätter eingeführt. Zwischen dem Fluidanschluss 211 und den Fluidauslässen 214 weist die innere Hülse aussenseitig einen zylindrischen Hauptabschnitt auf, der in distaler Richtung aussenseitig von einer kurzen ringförmigen Verjüngung gefolgt ist. Diese Verjüngung ist wiederum von einer Spitze 213 gefolgt. Die Spitze 213 weist mehrere (hier vier) längs verlaufende, zum distalen Ende hin spitz zulaufende Stege auf, zwischen denen mehrere sich zum distalen Ende hin verjüngende, zur zentralen Längsachse hin geneigte

Gasauslasskanäle ausgebildet sind.

Die äussere Hülse 220 ist vom distalen Ende her auf die innere Hülse 210 aufgeschoben. Dabei greift eine Ringwulst 215 am Einlassbereich der inneren Hülse 210 derart in eine komplementäre Ringnut in der Begrenzungswand der äusseren Hülse 220 ein, dass zum proximalen Ende des Sprühkopfes hin eine gasdichte Verbindung zwischen der inneren und der äusseren Hülse gewährleistet ist. Im Bereich des Hauptabschnitts der inneren Hülse verläuft die äussere Hülse 220 zumindest teilweise radial beabstandet zur inneren Hülse 210 und bildet zusammen mit der inneren Hülse einen oder mehrere längs verlaufende Gasverteilkanäle 223. Im Bereich des distalen Endes des Sprühkopfes enden die Gasverteilkanäle 223 bei der ringförmigen Verjüngung der inneren Hülse, die zusammen mit der äusseren Hülse in diesem Bereich einen Ringraum begrenzt.

Die äussere Hülse weist an ihrem distalen Ende eine zentrale Öffnung auf, die sich in distaler Richtung konisch verjüngt. Der diese Öffnung begrenzende Wandbereich der äusseren Hülse liegt auf den Stegen der Spitze 213 auf und begrenzt dadurch die zwischen den Stegen ausgebildeten Gasauslasskanäle zur radialen Aussenseite hin. Die Gasauslasskanäle verbinden den Ringraum mit mehreren Gasauslässen 224, die leicht zurückversetzt von den Fluidauslässen 214 angeordnet sind und diese ringförmig umgeben.

An der äusseren Hülse 220 ist ein schräg zur Längsrichtung radial abstehender Gaseinlassstutzen 221 ausgebildet, der einen Druckgasanschluss bildet. Der Gaseinlassstutzen 221 begrenzt einen Gaszuführungskanal 222, der in die Gasverteilkanäle 223 mündet. Der Gaszuführungskanal 222 und die Gasverteilkanäle 223 bilden auf diese Weise gemeinsam einen Druckgaskanal, der vom Druckgasanschluss zu den Gasauslässen verläuft.

Zum Versprühen eines in der Spritze 100 aufgenommenen fluiden Produkts wird der Sprühkopf 200 zunächst mit dem Auslassbereich 106 der Spritze 100 verbunden, wie dies in den Figuren 1 bis 3 dargestellt ist. Zu diesem Zweck ist der Auslassbereich 106 der Spritze 100 als männlicher Lueranschluss mit einer starren Sicherungshülse 105 ausgebildet. Der Einlassbereich 211 des Sprühkopfes 200 ist entsprechend als weiblicher Lueranschluss mit einem kurzen Aussengewinde ausgebildet. Dadurch ist eine sichere

Verbindung zwischen der Spritze und dem Sprühkopf sichergestellt.

Auf den Gaseinlassstutzen 221 wird nun ein Druckgasschlauch aufgesteckt. Hierzu ist der Gaseinlassstutzen an seinem freien Ende als männlicher Lueranschluss mit einer drehbar angebrachten Sicherungshülse 225 ausgebildet. Durch den Gaszuführungskanal 222 wird nun ein Druckgas zugeführt und gelangt durch die Gasverteilkanäle 223 und den Ringraum zu den Gasauslässen 224, wo es in Form mehrerer feiner Gasstrahlen austritt. Durch Druck auf die Kolbenstange 103 wird nun das zu versprühende Produkt durch den zentralen Fluidkanal und den oder die Fluidauslässe 214 hindurch ausgetragen und durch die Gasstrahlen zerteilt und versprüht. Dabei dient das statische Mischelement 212 dazu, das Produkt vor seinem Austritt aus dem Sprühkopf zu durchmischen und zu homogenisieren.

Dieser grundsätzliche Aufbau des Sprühkopfes 200 ist ähnlich zum Aufbau des Sprühkopfes der WO 2010/048734, und für weitere Details wird auf jenes Dokument verwiesen, insbesondere in Hinblick auf mögliche Ausgestaltungen der Spitze 213. Jenes Dokument illustriert auch, wie ein Sprühkopf dieses grundsätzlichen Aufbaus abgewandelt werden kann, um zwei Komponenten separat zum Sprühkopf zuzuführen und mit oder ohne Vormischung zu versprühen. Selbstverständlich sind auch solche Ausführungsformen von der vorliegenden Erfindung umfasst.

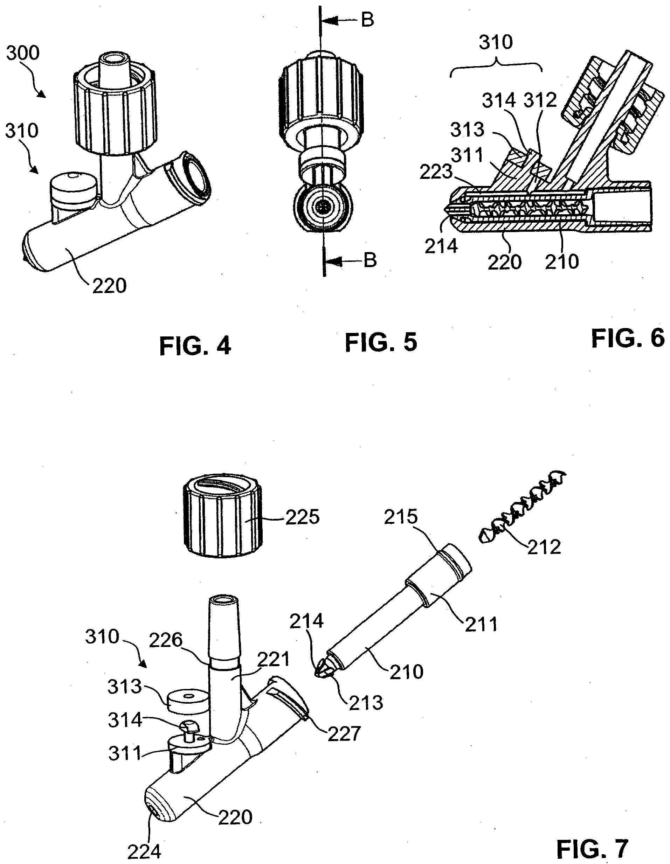

In den Figuren 4 bis 7 ist ein Sprühkopf 300 gemäss einem ersten Ausführungsbeispiel der vorliegenden Erfindung in verschiedenen Ansichten illustriert. Dieser Sprühkopf entspricht in Aufbau und Funktion sehr weitgehend dem Sprühkopf 200 der Figuren 1 bis 3, und gleich wirkende Teile sind daher mit den gleichen Bezugsziffern wie in den Figuren 1 bis 3 bezeichnet. Zusätzlich ist insbesondere in der Fig. 7 erkennbar, dass die Sicherungshülse 225 drehbar in einer Ringnut 226 des Gaszuführungsstutzens 221 gehalten ist; ausserdem ist insbesondere in der Fig. 4 und der Fig. 7 das Aussengewinde 227 erkennbar, mittels dessen der Sprühkopf in der Sicherungshülse 105 der Spritze 100 fixiert werden kann.

Um den Druck des Druckgases im Sprühkopf zu begrenzen, weist dieser Sprühkopf zusätzlich ein Überdruckventil 310 auf. Das Überdruckventil umfasst eine Ventilbasis 311, die an der äusseren Hülse 220 ausgebildet ist und in der dezentral ein Ventilauslasskanal 312 vorhanden ist. Dieser Ventilauslasskanal führt, ausgehend von den Gasverteilkanälen 223, schräg zur Längsrichtung radial nach aussen. Eine aus einem elastischen Material, z.B. Silikon, bestehende Ventilscheibe 313 verschliesst den Ventilauslasskanal 312 nach aussen hin. Dabei ist die Ventilscheibe 313 beabstandet zum Ventilauslasskanal 312 durch eine Haltestruktur 314 an der Ventilbasis 311 gehalten. Diese Haltestruktur umfasst einen zentralen, von der Ventilbasis 311 abstehenden Zapfen, der an seinem freien Ende hakenartig aufgeweitet ist.

Solange der Gasdruck in den Gasverteilkanälen 223 und damit auch im Ventilauslasskanal 312 einen bestimmten Schwellendruck nicht überschreitet, verschliesst die Ventilscheibe 313 den Ventilauslasskanal 312 in dichtender Weise. Sobald jedoch der Gasdruck den Schwellendruck überschreitet, übt der Gasdruck eine derartig grosse Kraft auf die Ventilscheibe 313 auf, dass sich diese elastisch verformt und den Ventilauslasskanal 312 nach aussen hin freigibt. Dadurch strömt so lange Druckgas durch den Ventilauslasskanal aus, bis der Schwellendruck wieder unterschritten wird und die Ventilscheibe 313 den Ventilauslasskanal 312 wieder verschliesst.

Die Grösse des Schwellendrucks wird dabei durch mehrere Faktoren bestimmt. Hierzu gehören insbesondere die von der Ventilscheibe 313 überdeckte Querschnittsfläche des Ventilauslasskanals 312 sowie die Vorspannung, Geometrie und Materialeigenschaften der Ventilscheibe 313. Indem diese Grössen verändert werden, lässt sich der Schwellendruck in einem weiten Bereich einstellen.

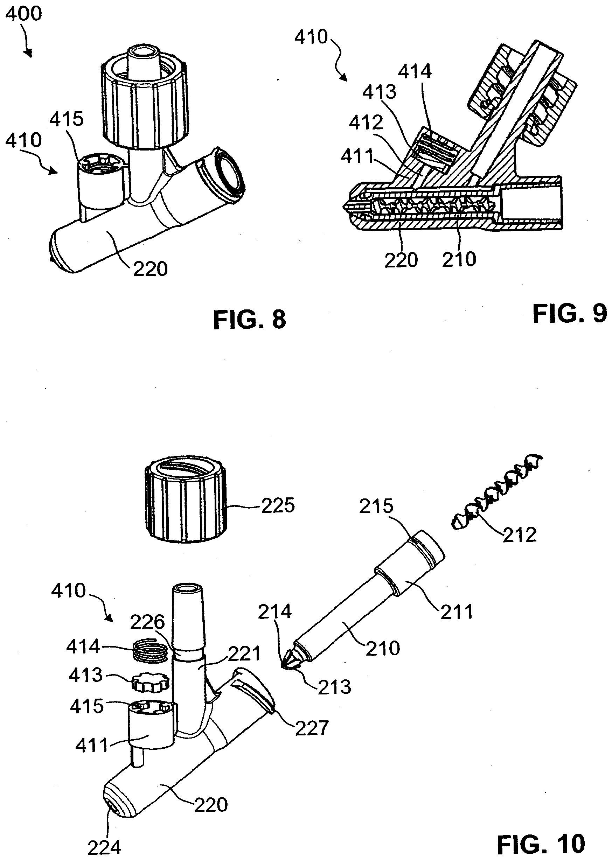

Ein Sprühkopf 400 gemäss einem zweiten Ausführungsbeispiel der vorliegenden Erfindung ist in den Figuren 8 bis 10 illustriert. Wiederum sind Aufbau und Funktion sehr ähnlich zum Sprühkopf 200 der Figuren 1 bis 3, und es werden wiederum die gleichen Bezugsziffern für gleich wirkende Teile verwendet.

Auch der Sprühkopf 400 weist ein Überdruckventil auf. Das Überdruckventil 410 umfasst eine Ventilbasis 411 mit einem zentralen Ventilauslasskanal 412. Dieser ist durch eine starre Ventilscheibe 413 verschlossen, die mittels einer Druckfeder 414 auf das freie Ende des Ventilauslasskanals 412 gepresst wird. Die Druckfeder ist mittels Haltenasen 415 in der Ventilbasis fixiert.

Auch bei diesem Ausführungsbeispiel verschliesst die Ventilscheibe 413 den Ventilauslasskanal 412, solange ein bestimmter Schwellendruck nicht überschritten wird. Sobald der Schwellendruck überschritten ist, führt die auf die Ventilscheibe 413 wirkende Druckkraft dazu, dass die Druckfeder 414 komprimiert wird, so dass die Ventilscheibe 413 den Ventilauslasskanal 412 freigibt. Wiederum kann auf diese Weise bei einem unerwünschten Druckanstieg so lange Druckgas entweichen, bis der Gasdruck den Schwellendruck wieder unterschreitet.

Der Schwellendruck lässt sich bei diesem Ausführungsbeispiel dadurch einstellen, dass die von der Ventilscheibe 413 überdeckte Querschnittsfläche des Ventilauslasskanals und/oder die Rückstellkraft der Druckfeder 414 variiert wird. Auch die Elastizität der Ventilscheibe hat Einfluss auf den Schwellendruck.

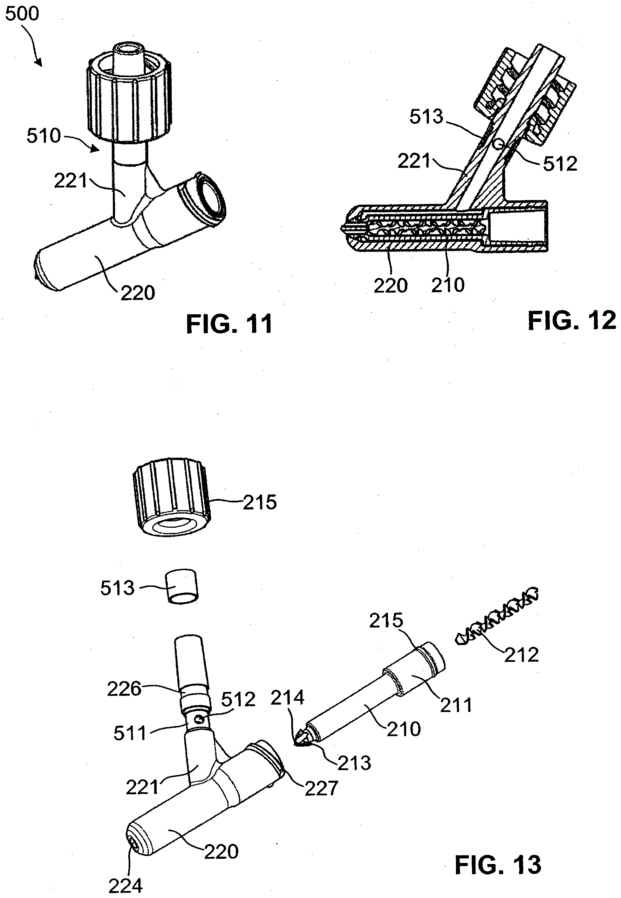

Ein Sprühkopf 500 gemäss einem dritten Ausführungsbeispiel der vorliegenden Erfindung ist in den Figuren 11 bis 13 dargestellt. Wiederum sind gleich wirkende Teile mit den gleichen Bezugszeichen wie in den Figuren 1 bis 3 bezeichnet. Der Sprühkopf 500 weist ein Überdruckventil im Bereich des Gaszuführungsstutzens 221 auf. Dazu ist auf der Aussenseite des Gaszuführungsstutzens 221 eine ringförmige Verjüngung 511 (siehe Fig. 13) ausgebildet. Im Bereich dieser Verjüngung weist die Begrenzungswand des Gaszuführungsstutzens 221 eine oder mehrere Öffnungen auf, die einen oder mehrere Ventilauslasskanäle 512 bilden. Auf die Verjüngung 511 ist eine hülsenförmige, elastische Ventilmanschette 513 aufgeschoben, die z.B. aus Silikon bestehen kann.

Solange ein Schwellendruck nicht überschritten wird, verschliesst die Manschette 513 die Ventilauslasskanäle 512. Sobald der Schwellendruck überschritten ist, reicht die Druckkraft des Druckgases aus, die Manschette 513 von der Verjüngung 511 abzuheben, so dass die Ventilauslasskanäle nach aussen hin freigegeben werden und Druckgas entweichen kann.

Bei diesem Ausführungsbeispiel lässt sich der Schwellendruck dadurch einstellen, dass die von der Manschette 513 überdeckte Öffnungsfläche und/oder die Materialeigenschaften und Dimensionen der Manschette verändert werden.

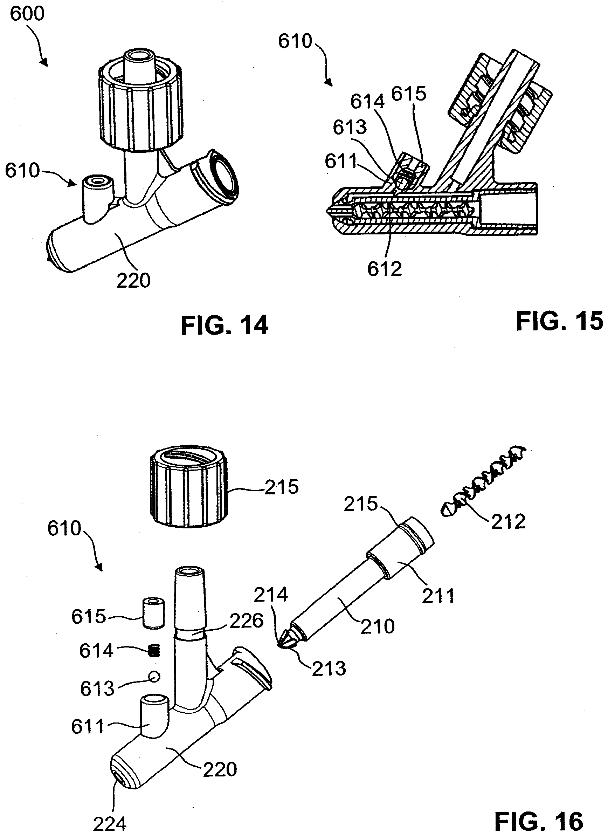

In den Figuren 14 bis 16 ist ein viertes Ausführungsbeispiel der vorliegenden Erfindung illustriert. Ein Sprühkopf 600 umfasst ein Überdruckventil 610 in Form eines Kugelventils. Ein Ventileinsatz 615 hält eine durch eine Druckfeder 614 belastete Ventilkugel 613 in einer Ventilbasis 611 derart, dass die Ventilkugel 613 einen Ventilauslasskanal 612 verschliesst. Bei Überschreiten eines Schwellendrucks wird die Ventilkugel 613 vom Ventilauslasskanal 612 abgehoben und gibt so den Ventilauslasskanal 612 nach aussen frei. Der Schwellendruck lässt sich hier durch geeignete Wahl der Dimensionen des Ventilauslasskanals 612 und der Rückstellkraft der Druckfeder 614 einstellen.

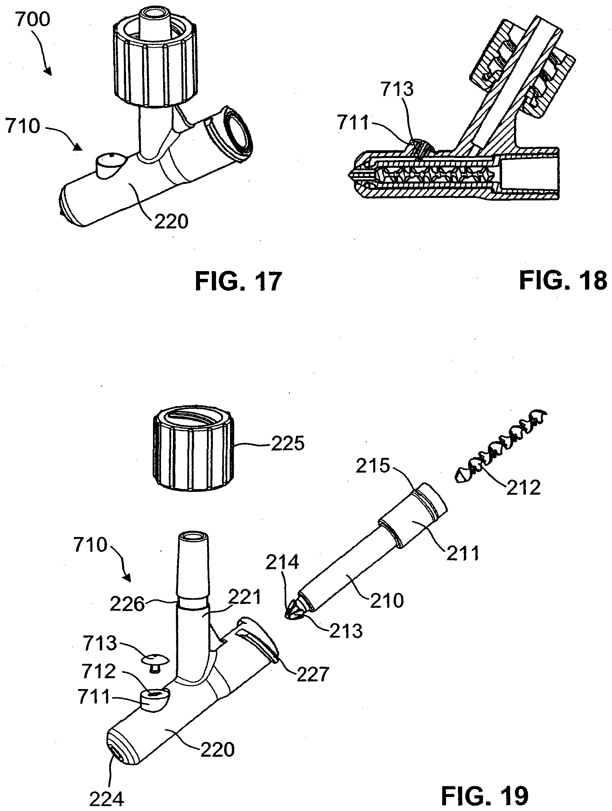

In den Figuren 17 bis 19 ist ein fünftes Ausführungsbeispiel der vorliegenden Erfindung illustriert. Der Sprühkopf 700 weist ein Überdruckventil 710 auf, das als sogenanntes Schirmventil (englisch "umbrella valve") oder Pilzventil ausgebildet ist. In einer Ventilbasis 711 ist ein zentraler Ventilauslasskanal 712 ausgebildet, der eine nicht rotationssymmetrische Form aufweist. In diesen Kanal 712 ist ein pilzförmiger Ventilkörper 713 aus einem elastischen Material, z.B. Silikon, eingesetzt. Ein schirmartiger oberer Bereich des Ventilkörpers 713 überdeckt den Ventilauslasskanal, solange ein Schwellendruck nicht überschritten wird. Bei Überschreiten des Schwellendrucks hebt die Druckkraft den schirmartigen Bereich des Ventilkörpers 713 vom Ventilauslasskanal ab, so dass das Druckgas aus dem Ventilauslasskanal entweichen kann. Der Schwellendruck lässt sich bei dieser Art von Ventil durch geeignete Dimensionierung des Ventilauslasskanals sowie durch geeignete Wahl der Vorspannung, der Dimensionen und des Materials des Ventilkörpers in weiten Bereichen einstellen.

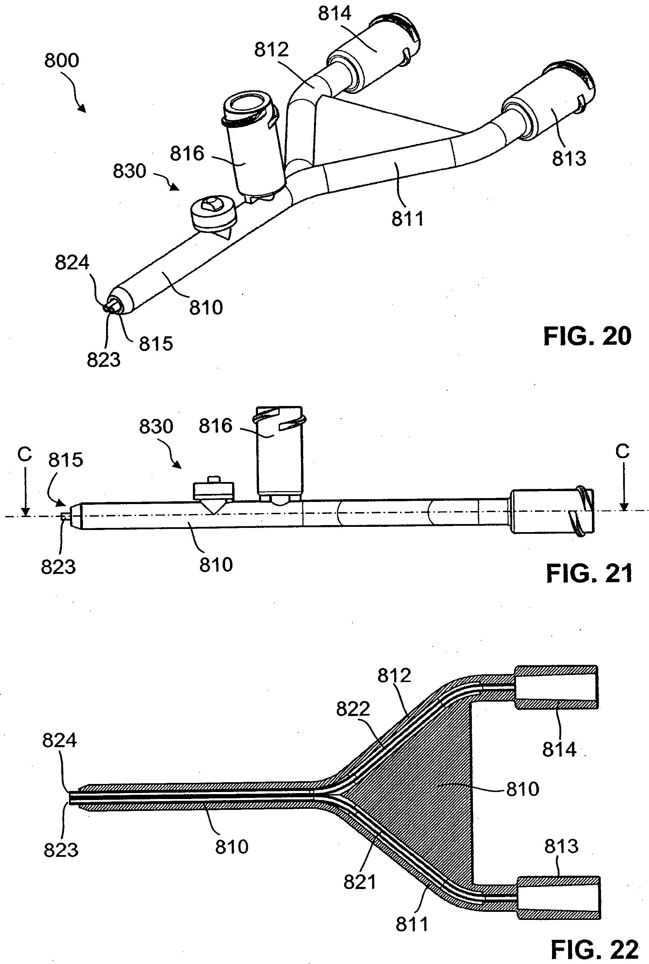

Ein sechstes Ausführungsbeispiel ist in den Figuren 20 bis 25 illustriert. Der hier dargestellte Sprühkopf 800 weist einen grundsätzlich anderen Aufbau als in den vorhergehenden Ausführungsbeispielen auf. Dieses Ausführungsbeispiel illustriert, dass die Erfindung an Sprühköpfen der unterschiedlichsten Bauformen verwirklicht werden kann.

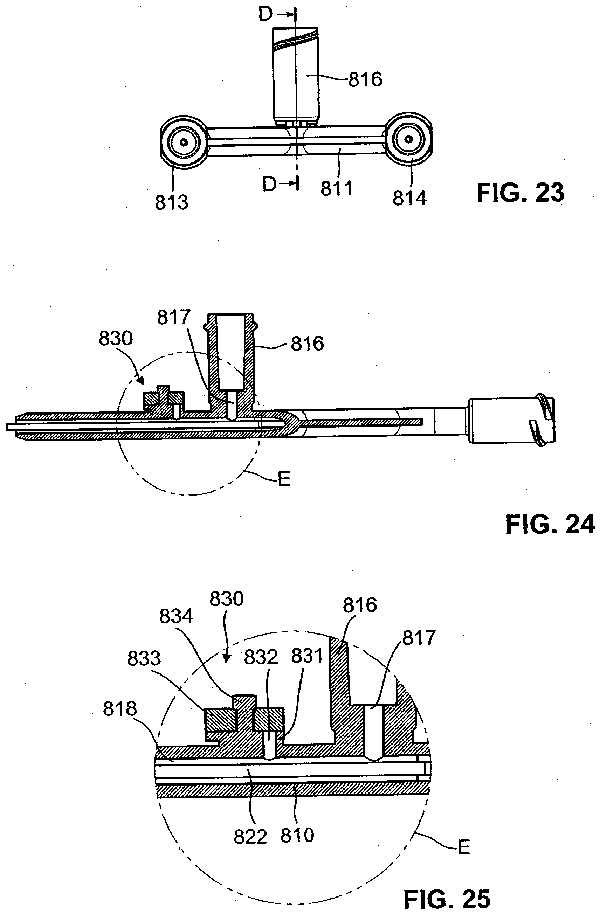

Der Sprühkopf 800 weist einen Y-förmigen Basiskörper auf, der zum distalen Ende des Sprühkopfes hin ein langgestrecktes, zylindrisches distales Rohr 810 bildet. Zum proximalen Ende des Sprühkopfes hin teilt sich dieses Rohr 810 in zwei gekrümmte Einlassrohre 811, 812 auf, die gemeinsam eine V-förmige Anordnung bilden. An ihren proximalen Enden sind diese Einlassrohre mit jeweils einem Fluidanschluss 813, 814 in Form eines weiblichen Lueranschlusses versehen. Von jedem dieser Fluidanschlüsse ausgehend erstreckt sich jeweils ein gebogenes Innenrohr 821, 822, z.B. aus Metall, oder ein flexibler Schlauch durch jeweils ein Einlassrohr 811, 812 und das distale Rohr 810 hindurch bin zum distalen Ende des Sprühkopfes, wo die Innenrohre an jeweils einem Fluidauslass 823, 824 enden; jedes dieser Innenrohre bildet auf diese Weise einen Fluidkanal, der sich von jeweils einem Fluidanschluss 813, 814 zu einem Fluidauslass 823, 824 am distalen Ende des Sprühkopfes erstreckt. Die Aussendurchmesser dieser beiden Innenrohre 821, 822 sind deutlich kleiner als der Innendurchmesser des distalen Rohrs 810. Der Zwischenraum zwischen den Innenrohren 821, 822 und der Begrenzungswand des distalen Rohrs 810 bildet auf diese Weise einen Gasverteilkanal 818 (siehe Figur 25).

Am Rohr 810 ist benachbart zur Stelle, an der die beiden Einlassrohre 811, 812 zusammentreffen, ein Druckgasanschluss 816 in Form eines weiblichen Lueranschlusses ausgebildet, der in einen Gaszuführungskanal 817 mündet. Dieser Gaszuführungskanal 817 verbindet den Druckgasanschluss mit dem Gasverteilkanal 818. Am distalen Ende des Sprühkopfes endet der Gasverteilkanal in einer Gasauslassöffnung 815, die leicht proximal zurückversetzt von den Fluidauslässen 823, 824 angeordnet ist, die durch die Enden der Innenrohre 821, 822 gebildet werden.

Dieser Sprühkopf ist insbesondere dazu geeignet, die Komponenten eines medizinischen Zweikomponentenklebers mit Hilfe eines Druckgases zu versprühen. Dazu wird an jeden der Fluidanschlüsse 813, 814 ein Fluidreservoir mit jeweils einer der zu versprühenden Komponenten angeschlossen. Insbesondere können z.B. die Auslässe einer Doppelspritze an die Fluidanschlüsse angeschlossen werden. An den Druckgasanschluss 816 wird ein Schlauch zur Zuführung eines Druckgases angeschlossen. Das Druckgas strömt durch den Gaszuführungskanal 817 und den Gasverteilkanal 818 zur Gasauslassöffnung 815 und erzeugt dadurch einen Gasstrom im Bereich der Fluidauslässe 823, 824. Nun trägt der Benutzer auf geeignete Weise die Komponenten aus den beiden Fluidreservoirs aus, so dass diese Komponenten durch die Innenrohre 821, 822 hindurch zu den Fluidauslässen 823, 824 gelangen und dort austreten. Im Falle einer Doppelspritze erfolgt dieser Austrag z.B. durch manuellen Druck auf die miteinander verbundenen Kolbenstangen der Doppelspritze. Sobald die Komponenten aus den Fluidauslässen 823, 824 austreten, werden sie vom Gasstrom in feine Tröpfchen zerteilt und treffen in Form eines feinen Sprühnebels auf einen Applikationsbereich auf.

Nahe des proximalen Endes des Rohrs 810 ist ein Überdruckventil 830 ausgebildet, welches im Wesentlichen gleich aufgebaut ist wie das Überdruckventil des ersten Ausführungsbeispiels (Figuren 4 bis 7). Insbesondere ist auch hier eine Ventilbasis 831 mit einem dezentralen Ventilauslasskanal 832 vorhanden. Eine elastische Ventilscheibe 833, die von einer zentralen Haltestruktur 834 an der Ventilbasis 831 gehalten ist, verschliesst den Ventilauslasskanal 832 nach aussen hin. Bei Überschreiten eines Schwellendrucks wird die Ventilscheibe entgegen ihrer elastischen Rückstellkraft vom Ventilauslasskanal 832 abgehoben und gibt diesen dadurch frei. Auch bei diesem Ausführungsbeispiel kann dadurch wirksam die Entstehung eines schädlichen Überdrucks vermieden werden.

Wie aus den vorstehenden Ausführungsbeispielen klar erkenntlich ist, ist die Erfindung keineswegs auf eine bestimmte Bauform eines Sprühkopfes beschränkt. Die Erfindung lässt sich an jedem an sich bekannten Sprühkopf verwirklichen, der über einen oder mehrere Anschlüsse für eines oder mehrere Fluidreservoirs sowie über einen Druckgasanschluss verfügt. Die Erfindung ist dabei weitgehend unabhängig von der spezifischen Topologie des Sprühkopfes. Sie lässt sich selbst an solchen Sprühköpfen verwirklichen, bei denen das Druckgas durch einen zentralen Druckgaskanal im Sprühkopf geführt wird, während die zu versprühenden Komponenten in einem den Druckgaskanal umgebenden Bereich geführt werden, wie dies z.B. beim Sprühkopf der US 5,605,541 der Fall ist, oder in denen die zu versprühenden Komponenten in einen Mischkammer abgegeben werden, zu der das Druckgas zentral zugeführt wird, wie dies z.B. in US 2003/0209612 der Fall ist. In diesem Fall kann es sinnvoll sein, das Überdruckventil im Bereich des Druckgasanschlusses vorzusehen, bevor das Druckgas in den zentralen Druckgaskanal eintritt.

Aus den vorstehenden Ausführungsbeispielen wird auch offensichtlich, dass die Erfindung keineswegs auf eine bestimmte Art eines Überdruckventils beschränkt ist. Während die vorstehend explizit beschriebenen Ventile aufgrund ihres einfachen Aufbaus und ihrer kostengünstigen Herstellung bevorzugt sind, können auch andere Arten von Überdruckventilen, die bei Überschreiten eines bestimmten Schwellendrucks öffnen, eingesetzt werden. Derartige Überdruckventile sind in einer grossen Zahl unterschiedlichster Ausgestaltungen aus dem Stand der Technik bekannt.

Während die vorstehend dargestellten Sprühköpfe jeweils eine verhältnismässig kurze Bauform haben, ist die Erfindung keineswegs auf derartig kurze Bauformen beschränkt. Die Erfindung lässt sich insbesondere auch mit Sprühköpfen einsetzen, die für endoskopische bzw. minimalinvasive Anwendungen bestimmt sind und aus diesem Grund in einem distalen Bereich eine langgestreckte, rohrförmige Bauform aufweisen. Bezüglich derartiger Bauformen sei z.B. auf US 5,810,885, US 2009/0209916 oder US 2009/0199848 verwiesen. Bei derartigen Sprühköpfen ist es bevorzugt, wenn das Überdruckventil in einem proximalen Bereich, stromaufwärts vom langgestreckt-rohrförmigen distalen Bereich, vorgesehen ist.

**BEZUGSZEICHENLISTE**

| | | | |
|---|---|---|---|
| 100 | Spritze | 412 | Ventilauslasskanal |
| 101 | Spritzenkörper | 413 | Ventilscheibe |
| 102 | Halteflansch | 414 | Druckfeder |
| 103 | Kolbenstange | 415 | Haltenase |
| 104 | Daumenauflage | 500 | Sprühkopf |
| 105 | Sicherungshülse | 510 | Überdruckventil |
| 106 | Auslassbereich | 511 | Verjüngung |
| 107 | Kolben | 512 | Ventilauslasskanal |
| 200 | Sprühkopf | 513 | Manschette |
| 210 | innere Hülse | 600 | Sprühkopf |
| 211 | Fluidanschluss | 610 | Überdruckventil |
| 212 | Mischelement | 611 | Ventilbasis |
| 213 | Spitze | 612 | Ventilauslasskanal |
| 214 | Fluidauslass | 613 | Ventilkugel |
| 215 | Ringwulst | 614 | Druckfeder |
| 216 | Fluidkanal | 615 | Ventileinsatz |
| 220 | äussere Hülse | 700 | Sprühkopf |
| 221 | Gaseinlassstutzen | 710 | Überdruckventil |
| 222 | Gaszuführungskanal | 711 | Ventilbasis |
| 223 | Gasverteilkanal | 712 | Ventilauslasskanal |
| 224 | Gasauslass | 713 | Verschlusskörper |
| 225 | Sicherungshülse | 800 | Sprühkopf |
| 300 | Sprühkopf | 810 | Grundkörper |
| 310 | Überdruckventil | 811 | erstes Einlassrohr |
| 311 | Ventilbasis | 812 | zweites Einlassrohr |
| 312 | Ventilauslasskanal | 813 | erster Einlassbereich |
| 313 | Ventilscheibe | 814 | zweiter Einlassbereich |
| 314 | Haltestruktur | 815 | Gasauslassöffnung |
| 400 | Sprühkopf | 816 | Druckgasanschluss |
| 410 | Überdruckventil | 817 | Gaszuführungskanal |
| 411 | Ventilbasis | 818 | Gasverteilkanal |
| 821 | erstes Innenrohr | 831 | Ventilbasis |
| 822 | zweites Innenrohr | 832 | Ventilauslasskanal |
| 823 | erster Fluidauslass | 833 | Verschlusskörper |
| 824 | zweiter Fluidauslass | 834 | Haltestruktur |
| 830 | Überdruckventil | | |

## Patentansprüche

1. Sprühkopf (300; 400; 500; 600; 700; 800) zum Versprühen mindestens einer fluiden Substanz unter Einsatz eines Druckgases, aufweisend:
mindestens einen Fluidanschluss (211; 813, 814), um den Sprühkopf mit mindestens einem Fluidreservoir für die zu versprühende Substanz zu verbinden;
mindestens einen Fluidkanal (216; 821; 822), der den Fluidanschluss (211; 813, 814) mit mindestens einem Fluidauslass (214; 823, 824) verbindet;
einen Druckgasanschluss (221; 816), um den Sprühkopf mit einer Druckgasquelle zu verbinden; und
mindestens einen Druckgaskanal (222, 223; 817, 818), der den Druckgasanschluss (221; 816) mit mindestens einem Druckgasauslass (224; 815) verbindet,
wobei der Fluidauslass (214; 823, 824) und der Druckgasauslass (224; 815) derart zueinander angeordnet sind, dass sich im Betrieb aus dem Druckgasauslass (224; 815) austretendes Druckgas mit einer aus dem Fluidauslass (214; 823, 824) austretenden Substanz vermischt, um die Substanz zu versprühen,
**dadurch gekennzeichnet, dass**
der Sprühkopf ein Überdruckventil (310; 410; 510; 610; 710; 830) aufweist, welches dazu ausgebildet ist, Druckgas aus dem Druckgaskanal (222, 223; 817, 818) abzuleiten, wenn das Druckgas einen Gasdruck aufweist, der einen Schwellendruck überschreitet, wobei das Überdruckventil (310; 410; 510; 610; 710; 830) direkt am Sprühkopf selbst angeordnet ist.

2. Sprühkopf nach Anspruch 1, wobei das Überdruckventil (310; 410; 510; 610; 710; 830) an einem Bereich des Druckgaskanals (222, 223; 817, 818) angeordnet ist, der bezüglich der Strömungsrichtung des Druckgases stromaufwärts vom Druckgasauslass (224; 815) angeordnet ist.

3. Sprühkopf nach Anspruch 1 oder 2, wobei das Überdruckventil (310; 410; 510; 610; 710; 830) an einem Bereich des Druckgaskanals (222, 223; 817, 818) angeordnet ist, der bezüglich der Strömungsrichtung des Druckgases stromabwärts vom Druckgasanschluss (221; 816) angeordnet ist.

4. Sprühkopf nach einem der vorhergehenden Ansprüche, wobei das Überdruckventil (310; 410; 610; 710; 830) an einem Bereich des Druckgaskanals (222, 223; 817, 818) vorgesehen ist, der den Fluidkanal (216; 821; 822) wenigstens teilweise umgibt.

5. Sprühkopf nach einem der vorhergehenden Ansprüche, wobei der Sprühkopf ein statisches Mischelement (212) aufweist, das im Fluidkanal (216) angeordnet ist.

6. Sprühkopf nach Anspruch 5, wobei das Überdruckventil (310; 410; 610; 710) an einem Bereich (223) des Druckgaskanals (222, 223) vorgesehen ist, der denjenigen Bereich des Fluidkanals (216) wenigstens teilweise umgibt, in dem das statische Mischelement (212) angeordnet ist.

7. Sprühkopf nach einem der vorhergehenden Ansprüche, wobei der Sprühkopf einen Basiskörper (210, 220; 810, 811, 812) und einen vom Basiskörper abstehenden Gaszufuhrstutzen aufweist, an dem der Druckgasanschluss (221; 816) ausgebildet ist, und wobei das Überdruckventil (310; 410; 610; 710; 830) am Basiskörper ausgebildet ist und einen Ventilauslasskanal (312; 412; 612; 712; 832) aufweist, der im Wesentlichen parallel zum Gaszufuhrstutzen verläuft.

8. Sprühkopf nach einem der vorhergehenden Ansprüche, wobei der Sprühkopf eine innere Hülse (210) aufweist, in welcher der mindestens eine Fluidkanal (226) ausgebildet ist, und eine äussere Hülse (220) aufweist, die die innere Hülse mindestens teilweise umgibt, wobei die äussere Hülse (220) und die innere Hülse (210) gemeinsam zumindest einen Abschnitt (223) des Druckgaskanals (222, 223) begrenzen, und wobei sowohl der Druckgasanschluss (221) als auch das Überdruckventil (310; 410; 610; 710) an der äusseren Hülse ausgebildet sind.

9. Sprühkopf nach Anspruch 8, wobei die äussere Hülse eine Längsachse definiert, und wobei das Überdruckventil (310; 410; 610; 710; 830) einen Ventilauslasskanal aufweist, dessen Längsrichtung zur Längsachse der äusseren Hülse 220 einen Winkel zwischen 30° und 90° einnimmt.

10. Sprühkopf nach einem der Ansprüche 1-3, wobei der Sprühkopf einen Basiskörper (210, 220; 810, 811, 812) und einen vom Basiskörper abstehenden Gaszufuhrstutzen aufweist, an dem der Druckgasanschluss (221; 816) ausgebildet ist, und wobei das Überdruckventil (510) am Gaszufuhrstutzen angeordnet ist.

11. Sprühkopf nach einem der vorhergehenden Ansprüche,
wobei der Sprühkopf einen ersten Fluidanschluss (813) und einen zweiten Fluidanschluss (814) aufweist, wobei jeder der Fluidanschlüsse dazu ausgebildet ist, den Sprühkopf mit jeweils einem Fluidreservoir für eine zu versprühende Komponente zu verbinden,
wobei der Sprühkopf einen ersten Fluidkanal (821) aufweist, der den ersten Fluidanschluss (813) mit einem ersten Fluidauslass (823) verbindet,
wobei der Sprühkopf einen zweiten Fluidkanal (822) aufweist, der den zweiten Fluidanschluss (814) mit einem zweiten Fluidauslass (824) verbindet, und
wobei der erste Fluidauslass (823), der zweite Fluidauslass (824) und der Druckgasauslass (815) derart zueinander angeordnet sind, dass sich im Betrieb aus dem Druckgasauslass (224; 815) austretendes Druckgas mit den beiden aus den Fluidauslässen (823, 824) austretenden Komponenten vermischt, um die Komponenten gemeinsam zu versprühen.

12. Sprühkopf nach einem der vorhergehenden Ansprüche, wobei das Überdruckventil (310; 710) umfasst:
einen elastischen Verschlusskörper (313; 713), der derart an einer Ventilbasis (311; 711) befestigt ist, dass er einen Ventilauslasskanal (312; 712) aufgrund seiner eigenen Elastizität verschliesst, solange der Gasdruck den Schwellendruck nicht überschreitet; oder
einen starren, beweglichen Verschlusskörper (413; 613), der derart federbelastet ist, dass er einen Ventilauslasskanal (412; 612) verschliesst, solange der Gasdruck den Schwellendruck nicht überschreitet; oder
eine elastische Manschette (513), die einen rohrförmigen Abschnitt des Sprühkopfes aussenseitig umgibt und aufgrund ihrer Elastizität mindestens einen im rohrförmigen Abschnitt ausgebildeten Ventilauslasskanal (512) verschliesst, solange der Gasdruck den Schwellendruck nicht überschreitet.

13. Sprühvorrichtung zum Versprühen eines medizinischen Klebemittels, aufweisend:
mindestens ein Fluidreservoir mit einem darin aufgenommenen medizinischen Klebemittel;
einen mit dem Fluidreservoir verbindbaren Sprühkopf nach einem der vorhergehenden Ansprüche; und
eine Austrageinrichtung zum Austragen des Klebemittels durch den Sprühkopf hindurch.

14. Sprühvorrichtung zum Versprühen von mindestens zwei Komponenten eines medizinischen Klebemittels, aufweisend:
mindestens zwei Fluidreservoirs mit jeweils einer darin aufgenommenen Komponente des medizinischen Klebemittels;
einen mit den Reservoirs verbindbaren Sprühkopf nach einem der vorhergehenden Ansprüche, wobei der Sprühkopf mindestens zwei Fluidanschlüsse zum Verbinden des Sprühkopfs mit jeweils einem der Fluidreservoirs und mindestens zwei Fluidkanäle aufweist, die die Fluidanschlüsse mit Fluidauslässen verbinden; und
eine Austrageinrichtung zum Austragen der Komponenten durch den Sprühkopf hindurch.

15. Verwendung eines Sprühkopfes nach einem der Ansprüche 1-12 oder einer Sprühvorrichtung nach Anspruch 13 oder 14 zum Versprühen eines medizinischen Produkts oder von Komponenten eines medizinischen Produkts, insbesondere eines medizinischen Fibrinklebers.

## Claims

1. A spray head (300; 400; 500; 600; 700; 800) for spraying at least one fluid substance with the use of compressed gas, comprising:
at least one fluid connector (211; 813, 814) in order to connect the spray head to at least one fluid reservoir for the substance to be sprayed;
at least one fluid channel (216; 821; 822) that connects the fluid connector (211; 813, 814) to at least one fluid outlet (214; 823, 824);
a compressed gas connector (221; 816) in order to connect the spray head to a source of compressed gas; and
at least one compressed gas channel (222, 223; 817, 818) that connects the compressed gas connector (221; 816) to at least one compressed gas outlet (224; 815),
wherein the fluid outlet (214; 823, 824) and the compressed gas outlet (224; 815) are arranged relative to each other in such a manner that during operation compressed gas exiting the compressed gas outlet (224; 815) mixes with a substance exiting the fluid outlet (214; 823, 824) in order to spray the substance,
**characterised in that**
the spray head comprises a pressure relief valve (310; 410; 510; 610; 710; 830) that is configured to discharge compressed gas from the compressed gas channel (222, 223; 817, 818) when the compressed gas has a gas pressure that exceeds a threshold pressure, wherein the pressure relief valve (310; 410; 510; 610; 710; 830) is directly provided on the spray head itself.

2. The spray head according to claim 1, wherein the pressure relief valve (310; 410; 510; 610; 710; 830) is arranged in a region of the compressed gas channel (222, 223; 817, 818), which region in terms of the direction of flow of the compressed gas is arranged upstream of the compressed gas outlet (224; 815).

3. The spray head according to claim 1 or 2, wherein the pressure relief valve (310; 410; 510; 610; 710; 830) is arranged in a region of the compressed gas channel (222, 223; 817, 818), which region in terms of the direction of flow of the compressed gas is arranged downstream of the compressed gas connector (221; 816).

4. The spray head according to any one of the preceding claims, wherein the pressure relief valve (310; 410; 610; 710; 830) is arranged in a region of the compressed gas channel (222, 223; 817, 818), which region at least partly encloses the fluid channel (216; 821; 822).

5. The spray head according to any one of the preceding claims, wherein the spray head comprises a static mixing element (212) that is arranged in the fluid channel (216).

6. The spray head according to claim 5, wherein the pressure relief valve (310; 410; 610; 710) is provided in a region (223) of the compressed gas channel (222, 223), which region (223) at least partly encloses the region of the fluid channel (216), in which region the static mixing element (212) is arranged.

7. The spray head according to any one of the preceding claims, wherein the spray head comprises a basic body (210, 220; 810, 811, 812) and a gas feed pipe that protrudes from the basic body, on which gas feed pipe the compressed gas connector (221; 816) is provided, and wherein the pressure relief valve (310; 410; 610; 710; 830) is provided on the basic body and comprises a valve outlet channel (312; 412; 612; 712; 832) that extends so as to be essentially parallel to the gas feed pipe.

8. The spray head according to any one of the preceding claims, wherein the spray head comprises an inner sleeve (210) in which the at least one fluid channel (226) is formed, and an outer sleeve (220) that at least partly encloses the inner sleeve, wherein the outer sleeve (220) and the inner sleeve (210) together delimit at least one section (223) of the compressed gas channel (222, 223), and wherein both the compressed gas connector (221) and the pressure relief valve (310; 410; 610; 710) are formed on the outer sleeve.

9. The spray head according to claim 8, wherein the outer sleeve defines a longitudinal axis, and wherein the pressure relief valve (310; 410; 610; 710; 830) comprises a valve outlet channel whose longitudinal direction is arranged relative to the longitudinal axis of the outer sleeve 220 at an angle of between 30° and 90°.

10. The spray head according to any one of claims 1-3, wherein the spray head comprises a basic body (210, 220; 810, 811, 812) and a gas feed pipe that protrudes from the basic body, which gas feed pipe comprises the compressed gas connector (221; 816), and wherein the pressure relief valve (510) is arranged on the gas feed pipe.

11. The spray head according to any one of the preceding claims,
wherein the spray head comprises a first fluid connector (813) and a second fluid connector (814), wherein each of the fluid connectors is designed to connect the spray head with a respective fluid reservoir for a component to be sprayed,
wherein the spray head comprises a first fluid channel (821) that connects the first fluid connector (813) to a first fluid outlet (823),
wherein the spray head comprises a second fluid channel (822) that connects the second fluid connector (814) to a second fluid outlet (824), and
wherein the first fluid outlet (823), the second fluid outlet (824) and the compressed gas outlet (815) are arranged relative to each other in such a manner that during operation compressed gas exiting the compressed gas outlet (224; 815) mixes with the two components exiting the fluid outlets (823, 824) in order to spray both the components.

12. The spray head according to any one of the preceding claims, wherein the pressure relief valve (310; 710) comprises:
an elastic closure body (313; 713) that is attached to a valve base (311; 711) in such a manner that because of its intrinsic elasticity it closes a valve outlet channel (312; 712), provided the gas pressure does not exceed the threshold pressure; or
a rigid, movable closure body (413; 613) that is spring-loaded in such a manner that it closes a valve outlet channel (412; 612), provided the gas pressure does not exceed the threshold pressure; or
an elastic sleeve (513) that encloses a tubular section of the spray head on the outside, and due to its elasticity closes at least one valve outlet channel (512) formed in the tubular section, provided the gas pressure does not exceed the threshold pressure.

13. A spray device for spraying a medical adhesive, comprising:
at least one fluid reservoir with a medical adhesive contained therein;
a spray head according to any one of the preceding claims, which spray head is connectable to the fluid reservoir; and
a dispensing device for dispensing the adhesive through the spray head.

14. A spray device for spraying at least two components of a medical adhesive, comprising:
at least two fluid reservoirs, each comprising a component of the medical adhesive contained therein;
a spray head according to any one of the preceding claims, which spray head is adapted to be connected to the reservoirs, wherein the spray head comprises at least two fluid connectors for connecting the spray head in each case to one of the fluid reservoirs, and at least two fluid channels that connect the fluid connectors to fluid outlets; and
a dispensing device for dispensing the components through the spray head.

15. The use of a spray head according to any one of claims 1-12 or of a spray device according to claim 13 or 14 for spraying a medical product or components of a medical product, in particular a medical fibrin glue.

## Revendications

1. Une tête de pulvérisation (300 ; 400 ; 500 ; 600 ; 700 ; 800) destinée à pulvériser au moins une substance fluide par application d'un gaz comprimé, présentant :
au moins un raccordement de fluide (211 ; 813 ; 814), pour connecter la tête de pulvérisation à au moins un réservoir de fluide pour la substance destinée à être pulvérisée ;
au moins un canal de fluide (216 ; 821 ; 822), qui connecte le raccordement de fluide (211 ; 813 ; 814) à au moins une sortie de fluide (214 ; 823 ; 824) ;
un raccordement à gaz comprimé (221 ; 816), pour connecter la tête de pulvérisation à une source à gaz comprimé ; et
au moins un canal à gaz comprimé (222, 223 ; 817, 818), qui connecte le raccordement à gaz comprimé (221 ; 816) à au moins une sortie à gaz comprimé (224 ; 815),
où la sortie de fluide (214 ; 823 ; 824) et la sortie à gaz comprimé (224 ; 815) sont disposées l'une par rapport à l'autre de telle manière que lors du fonctionnement, le gaz comprimé sortant de la sortie à gaz comprimé (224 ; 815) est mélangé avec une substance sortant de la sortie de fluide (214 ; 823 ; 824), pour pulvériser la substance,
**caractérisé en ce que**
la tête de pulvérisation dispose d'une soupape de surpression (310 ; 410 ; 510 ; 610 ;710 ; 830), laquelle est formée de sorte à évacuer le gaz comprimé hors du canal à gaz comprimé (222, 223, 817, 818), lorsque le gaz comprimé présente un pression de gaz qui dépasse une pression seuil, où la soupape de surpression (310 ; 410 ; 510; 610;710; 830) est disposée directement sur la tête de pulvérisation elle-même.

2. La tête de pulvérisation selon la revendication 1, où la soupape de surpression (310, 410, 510, 610, 710, 830) est disposée au niveau d'un domaine du canal à gaz comprimé (222, 223, 817, 818) qui est disposé en amont de la sortie à gaz comprimé (224; 815) par rapport à la direction d'écoulement du gaz comprimé.

3. La tête de pulvérisation selon la revendication 1 ou 2, où la soupape de surpression (310, 410, 510, 610, 710, 830) est disposée au niveau d'un domaine du canal à gaz comprimé (222, 223, 817, 818) qui est disposé en aval du raccordement à gaz comprimé (221; 816) par rapport à la direction d'écoulement du gaz comprimé.

4. La tête de pulvérisation selon une quelconque des revendications précédentes, où la soupape de surpression (310, 410, 610, 710, 830) est prévue au niveau d'un domaine du canal à gaz comprimé (222, 223, 817, 818) qui entoure par au moins partiellement le canal de fluide (216; 821; 822).

5. La tête de pulvérisation selon une quelconque des revendications précédentes, où la tête de pulvérisation comprend un élément mélangeur (212) statique, qui est disposé dans le canal de fluide (216).

6. La tête de pulvérisation selon la revendication 5, où la soupape de surpression (310, 410, 610, 710) est prévue au niveau d'un domaine (223) du canal à gaz comprimé (222, 223) qui entoure au moins partiellement le domaine du canal de fluide (216) dans lequel l'élément mélangeur (212) statique est disposé.

7. La tête de pulvérisation selon une quelconque des revendications précédentes, où la tête de pulvérisation présente un corps de base (210, 220; 810, 811, 812) et un embout d'alimentation en gaz s'étendant à partir du corps de base, au niveau duquel le raccordement à gaz comprimé (221; 816) est formé, et où la soupape de surpression (310, 410, 610, 710, 830) est formée au niveau du corps de base et présente un canal de sortie de soupape (312; 412; 612; 712; 832) qui s'étend essentiellement en parallèle à l'embout d'alimentation en gaz.

8. La tête de pulvérisation selon une quelconque des revendications précédentes, où la tête de pulvérisation présente une gaine intérieure (210), dans laquelle l'au moins un canal de fluide (226) est formé, et une gaine extérieure (220) qui entoure au moins partiellement la gaine intérieure, où la gaine extérieure (220) et la gaine intérieure (210) limitent conjointement au moins une section (223) du canal à gaz comprimé (222, 223) et où le raccordement à gaz comprimé (221) ainsi que la soupape de surpression (310, 410, 610, 710) sont formées au niveau de la gaine extérieure.

9. La tête de pulvérisation selon la revendication 8, où la gaine extérieure définit un axe longitudinal, et où la soupape de surpression (310, 410, 610, 710, 830) présente un canal de sortie de soupape, dont la direction longitudinale occupé un angle entre 30° et 90° par rapport à l'axe longitudinal de la gaine extérieure (220).

10. La tête de pulvérisation selon une quelconque des revendications 1 à 3, où la tête de pulvérisation présente un corps de base (210, 220; 810, 811, 812) et un embout d'alimentation en gaz s'étendant à partir du corps de base, au niveau duquel un raccordement à gaz comprimé (221; 816) est formé, et où la soupape de surpression (510) est disposée au niveau de l'embout d'alimentation en gaz.

11. La tête de pulvérisation selon une des revendications précédentes, où la tête de pulvérisation présente un premier raccordement de fluide (813) et un deuxième raccordement de fluide (814), où chacun des raccordements de fluide est formé de sorte que la tête de pulvérisation est connectée à respectivement un réservoir de fluide pour un composant destiné à être pulvérisé, où la tête de pulvérisation présente un premier canal de fluide (821) qui connecte le premier raccordement de fluide (813) avec une première sortie de fluide (823), où la tête de pulvérisation présente un deuxième canal de fluide (822) qui connecte le deuxième raccordement de fluide (814) avec une deuxième sotie de fluide (824), et où la première sortie de fluide (823), la deuxième sortie de fluide (824) et la sortie à gaz comprimé (815) sont disposées de telle manière l'une par rapport à l'autre, que lors du fonctionnement, le gaz comprimé sortant de la sortie à gaz comprimé (224; 815) est mélangé avec les deux composants sortant des sorties de fluide (823, 824) pour pulvériser les composants conjointement.

12. La tête de pulvérisation selon une quelconque des revendications précédentes, où la soupape de surpression (310; 710) comprend:
un corps de fermeture élastique (313; 713), qui est fixé à une base de soupape (311; 711) de sorte qu'il ferme un canal de sortie de soupape (312; 712) à base de sa propre élasticité, tant que la pression de gaz ne dépasse pas une pression seuil; ou
un corps de fermeture rigide, mobile (413; 613) qui est commandé par un ressort de sorte qu'il ferme un canal de sortie de soupape (412; 612), tant que la pression de gaz ne dépasse pas la pression seuil; ou
une manchette élastique (513), qui entoure une section tubulaire de la tête de pulvérisation à l'extérieur et ferme à base de son élasticité au moins un canal de sortie de soupape (512) formé dans la section tubulaire, tant que la pression de gaz ne dépasse pas une pression seuil.

13. Un dispositif de pulvérisation pour la pulvérisation d'un adhésif médical, comprenant:
au moins un réservoir de fluide avec un adhésif médical disposé à son intérieur;
au moins une tête de pulvérisation capable d'être connectée au réservoir de fluide selon une quelconque des revendications précédentes; et
une installation de décharge pour la décharge de l'adhésif à travers la tête de pulvérisation.

14. Un dispositif de pulvérisation pour la pulvérisation d'au moins deux composants d'un adhésif médical, comprenant:
au moins deux réservoirs de fluide ayant respectivement un composant de l'adhésif médical disposé à leur intérieur;
une tête de pulvérisation capable d'être connecté avec les réservoirs selon une des revendications précédentes, où la tête de pulvérisation présente au moins deux raccordements de fluide pour une connexion de la tête de pulvérisation à respectivement un des réservoirs de fluide et au moins deux canaux de fluide qui connectent les raccordements de fluide avec les sorties de fluide; et
un dispositif de décharge pour la décharge des composants à travers la tête de pulvérisation.

15. L'utilisation d'une tête de pulvérisation selon une des revendications 1 à 12 ou d'un dispositif de pulvérisation selon la revendication 13 ou 14 pour la pulvérisation d'un produit médical ou des composants d'un produit médical, en particulier d'un adhésif médical à base de fibrine.
